# EUROPEAN PATENT APPLICATION

(11) **EP 2 372 380 A2**
(43) Date of publication of application: **05.10.2011**
(21) Application number: 11152057.3
(22) Date of filing: 25.01.2011
(51) Int. Cl.: G01R 31/36

(54) **Apparatus, system and method of managing a battery of a wirelessly communicable radiographic cassette**

(30) Priority: 25.03.2010 JP 2010070507
(71) Applicant: Fujifilm Corporation, Minato-ku Tokyo (JP)
(72) Inventor: Kamiya, Takeshi, Kanagawa (JP); Kitagawa, Yusuke, Kanagawa (JP)
(74) Representative: Höhfeld, Jochen

(57) **Abstract**

A current charge level (R) of a battery presently used for supplying a wirelessly communicable cassette (14) is detected and transmitted to a console (16) of an X-ray imaging apparatus (2). In the console, a power consumption calculator (70) calculates an estimated amount of power consumption (p) by the cassette for each of received orders for inspections. When a new order for inspection is received, or one of the ordered inspections is canceled or finished, or the battery of the cassette is replaced, the last inspection until which the presently used battery can effectively supply the cassette is determined among the ordered inspections on the basis of the detected current charge level (R) and the estimated power consumption amounts (p) for the individual inspections, to display a message prompting to replace the presently used battery before the end of the determined last inspection.

## Description

### BACKGROUND OF THE INVENTION

### 1. Field of the Invention

The present invention relates to an apparatus, system and method of managing battery level of a wirelessly communicable cassette for use in radiography.

### 2. Description of the Related Art

Radiography is widely used in medical field. For example, radioactive rays such as X-rays are projected onto an object or patient under inspection, and the rays passing through the patient body are detected to acquire images of internal structures of the patient. Conventionally, radiographic photosensitive film is exposed to radioactive rays to form an image thereon. Now replacing the radiographic film is a digital radiation detector, called imaging plate or flat panel detector, which can readily provide digitized radiological images on monitor screen.

As a radiographic equipment with improved operability, a cassette having a radiation detector and a wireless communication device incorporated therein is disclosed for example in JPA 2006-043191. This prior art also discloses a device for charging and replacing battery packs of the wirelessly communicable radiation detector cassette. The battery charging and replacing device calculates how much the cassette will consume the power for capturing a total number of images expected to be captured from candidates for inspection. The battery charging and replacing device also measures battery level of each cassette to compare it with the calculated power consumption. If the charge level of one battery pack is less than the estimated necessary power consumption, the battery pack must be replaced with another of a sufficient charge level. In addition, deterioration of the individual battery pack is checked by monitoring how often the battery pack has been charged. For the purpose of averaging the degree of deterioration of the battery packs, one that was used or charged least frequently is first selected to be loaded in the cassette, given priority over other battery packs.

Although it is not a wirelessly communicable cassette, a portable X-ray modality usable during making the rounds is disclosed in US Patent No . 7 , 3 09 , 159 and US Patent No. 7,643,613 (corresponding to JPA 2006-158508). This prior art describes a battery for supplying power to the portable X-ray modality, and a method of estimating power consumption from information about orders for inspections, and comparing the estimated power consumption with the battery level to check if the battery level is sufficient. The data of orders for inspections is entered by doctors in diagnosis and treatment departments at terminals of a hospital information system (HIS) and transmitted over a radiological information system (RIS) to a console of the radiography module. A radiologist may check the orders for inspections on a work list screen of a display of the console, to carry out the inspections as ordered.

Upon each entry of an inspection order, the work list on the screen is revised correspondingly. The work list is also revised when an inspection order is canceled or an ordered inspection is accomplished. Accordingly, the necessary power consumption will vary with the change of the requested inspections on the order list. The above-mentioned prior arts do not take account of such a change with time in power consumption. As a result, the battery can run down during an inspection that is added after the estimation and comparison of power consumption with the remaining charge of the loaded battery. In that case, the radiologist taking the inspection should replace the batteries, which will certainly bother or disgust the patient under the inspection.

In particular, as the battery for the wireless communication cassette is small in size for the sake of minimizing and lightening the cassette, the capacity of the battery is so small that it requires replacing relatively frequently. Therefore, there is a demand for a solution that enables replacing the batteries of the cassette at appropriate timing without any stress on patients under inspections.

### SUMMARY OF THE INVENTION

In view of the foregoing, the present invention has an object to enable replacing batteries at appropriate timing without applying any stress on patients under inspections.

In an aspect of the present invention, a battery managing apparatus for a wirelessly communicable cassette for use in inspections comprises a first battery level detector that detects a current charge level of a battery presently used in the cassette and a power consumption calculator that calculates an estimated amount of power consumption by the cassette for each of received orders for inspections.

On the basis of the current charge level detected by the first battery level detector and the power consumption calculated by the power consumption calculator, a battery level calculator calculates an estimated remaining charge level of the presently used battery, which will remain at the completion of each of the ordered inspections. On the basis of the estimated remaining charge level, a first judgment device determines the last inspection among the ordered inspections, until which the presently used battery can effectively supply the cassette. A first display device displays a time limit to replace the presently used battery at latest by the end of the last inspection until which the presently used battery can effectively supply the cassette.

The first battery level detector, the power consumption calculator, the battery level calculator and the first judgment device are activated when the orders for inspections are revised, and/or every time an inspection on a patient is finished, and/or every time the battery of the cassette is replaced.

The battery level calculator calculates the estimated remaining charge level of the presently used battery by subtracting an integrated value of the individual power consumption amounts from the current charge level of the presently used battery.

The first judgment device compares the estimated remaining charge level with a threshold representative of a minimum effective battery level. Where the estimated remaining charge level gets less than the threshold, the first judgment device determines that the presently used battery will not be able to supply for the inspection about which the remaining charge level is estimated.

According to a preferred embodiment, the battery managing apparatus further comprises a second battery level detector that detects a current charge level of a spare battery other than the battery presently used in the cassette, and a second judgment device that determines based on the current charge level of the spare battery and the power consumption calculated by the power consumption calculator whether the spare battery is available for replacement with the presently used battery.

In this embodiment, information about an available spare battery may be displayed at latest by the end of the last inspection until which the presently used battery can supply the cassette effectively.

In one embodiment, the second judgment device determines that the spare battery is available for replacement with the presently used battery when the current charge level of the spare battery gets equal to or higher than a sum of an integrated value of the power consumption as calculated by the power consumption calculator and the threshold representative of the minimum effective battery level. The information about the available spare battery may be displayed when the second judgment device determines the available spare battery or immediately after the end of each inspection on one patient.

In another embodiment, the second judgment device determines the spare battery to be available for replacement when the current charge level of the spare battery is approximately equal to a sum of a power consumption amount necessary for one inspection during which the presently used battery will rundown and the threshold representative of the minimum effective battery level.

It may also be possible to calculate an estimated remaining charge level of the available spare battery, which will remain at the completion of each of the ordered inspections when the available spare battery is used therefor, on the basis of the current charge level of the available spare battery and the power consumption calculated by the power consumption calculator. Then, the first judgment device may determine, on the basis of the estimated remaining charge level of the available spare battery, the last inspection until which the available spare battery will be able to supply the cassette effectively. Thus, information about the last inspection until which the available spare battery will be able to supply the cassette effectively may be included in the information about the available spare battery.

In another aspect of the present invention, a method of managing batteries used for supplying wirelessly communicable cassettes comprises steps of detecting a current charge level of a battery presently used in the cassette; calculating an estimated amount of power consumption by the cassette for each of received orders for inspections; calculating an estimated remaining charge level of the presently used battery, which will remain at the completion of each of the ordered inspections, on the basis of the current charge level of the presently used battery and the estimated power consumption calculated for each inspection; determining on the basis of the estimated remaining charge level the last inspection among the ordered inspections, until which the presently used battery can effectively supply the cassette; and displaying a time limit to replace the presently used battery at latest by the end of the last inspection until which the presently used battery can effectively supply the cassette.

According to the present invention, the last inspection until which the current charge level of the presently used battery is sufficient for supplying the wirelessly communicable cassette is determined among the already-received ordered inspections. If the current charge level is determined insufficient for accomplishing all of the ordered inspections with the presently used battery, a time limit to replace the battery is displayed in advance. Therefore, the battery can be replaced at appropriate timing without any stress on patients under inspections.

### BRIEF DESCRIPTION OF THE DRAWINGS

The above and other objects and advantages of the present invention will be more apparent from the following detailed description of the preferred embodiments when read in connection with the accompanied drawings, wherein like reference numerals designate like or corresponding parts throughout the several views, and wherein:
Figure 1 is a diagram illustrating modalities, apparatuses and systems installed in a hospital;
Figure 2 is a diagram illustrating a flow of transmission of orders for inspection and a list of received orders;
Figure 3 is a perspective view of a wirelessly communicable cassette;
Figure 4 is a block diagram illustrating an electric structure of an X-ray imaging apparatus;
Figure 5 is a block diagram illustrating functional sections developed in a CPU of a console of the X-ray imaging apparatus;
Figure 6 is a graph illustrating a progress of battery level of the cassette estimated with respect to ordered inspections;
Figure 7 is a diagram illustrating an example of a display screen showing the time to replace the battery;
Figure 8 is a flowchart illustrating a sequence of processing for displaying the time to replace the battery;
Figure 9 is a diagram illustrating an embodiment which is configured to manage the usage of a plurality of batteries, including those being currently charged;
Figure 10 is a diagram illustrating an example of a display screen showing the time to replace the battery and an available alternative battery;
Figures 11A and 11B are diagrams illustrating examples of message windows informing of a recommendable alternative battery; and
Figure 12 is a diagram illustrating an example of a message window prompting to switch from wireless communication to wired communication.

### DETAILED DESCRIPTION OF THE PREFERRED EMBODIMENTS

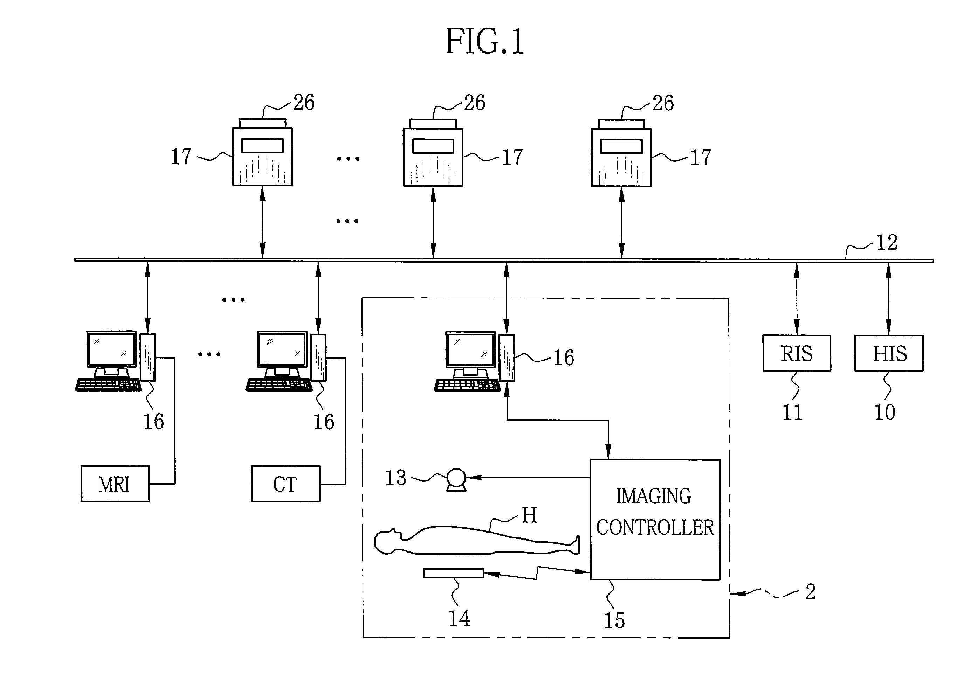

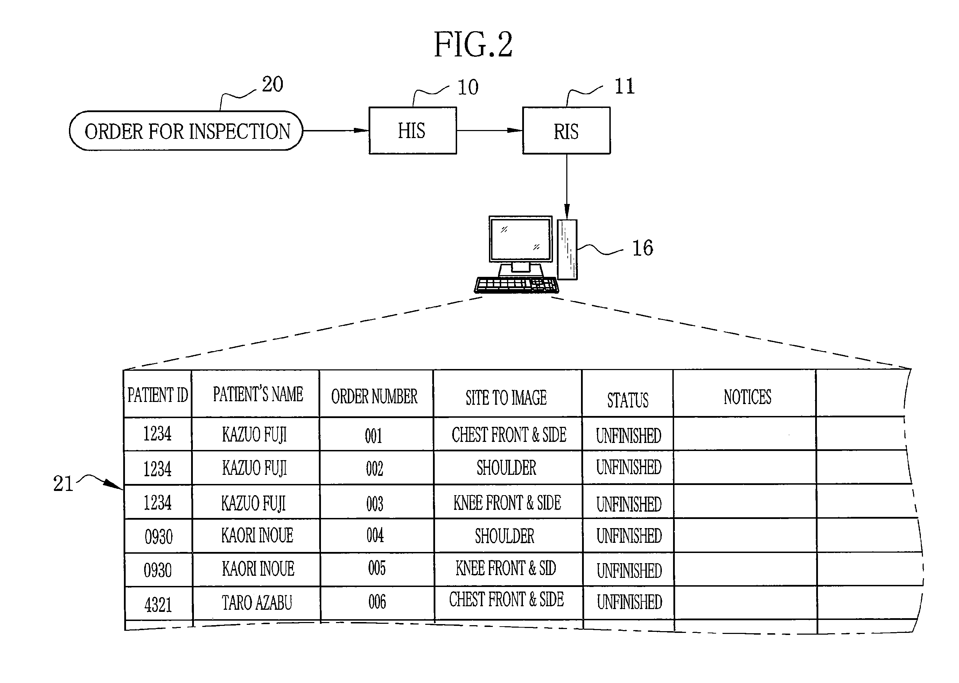

In Fig. 1, an X-ray imaging apparatus 2 may be installed in an X-ray room of a radiology department of a hospital, wherein a hospital information system (HIS) 10 serves for supervising a variety of medical information including electronic charts and appointments for inspections. In the radiology department, a radiology information system (RIS) 11 supervises operations of multiple modalities or medical imaging apparatuses including MRI and CT scanners and the X-ray radiography apparatus 2, as well as information about inspections including orders for inspections 20 (see Fig.2) and X-ray and other radiological images. These systems and modalities are interconnected through a local area network (LAN) 12 developed in the hospital.

As shown in Fig.2, a doctor in a diagnosis and treatment can submit an order for inspection 20 at a terminal of the HIS 10 to the radiology department, making an appointment for an inspection. Through the HIS 10, the order for inspection 20 is transferred to the RIS 11, which then delivers the inspection order 20 to a console 16 of an appropriate one of the X-ray imaging apparatus 2 and other modalities through the LAN 12.

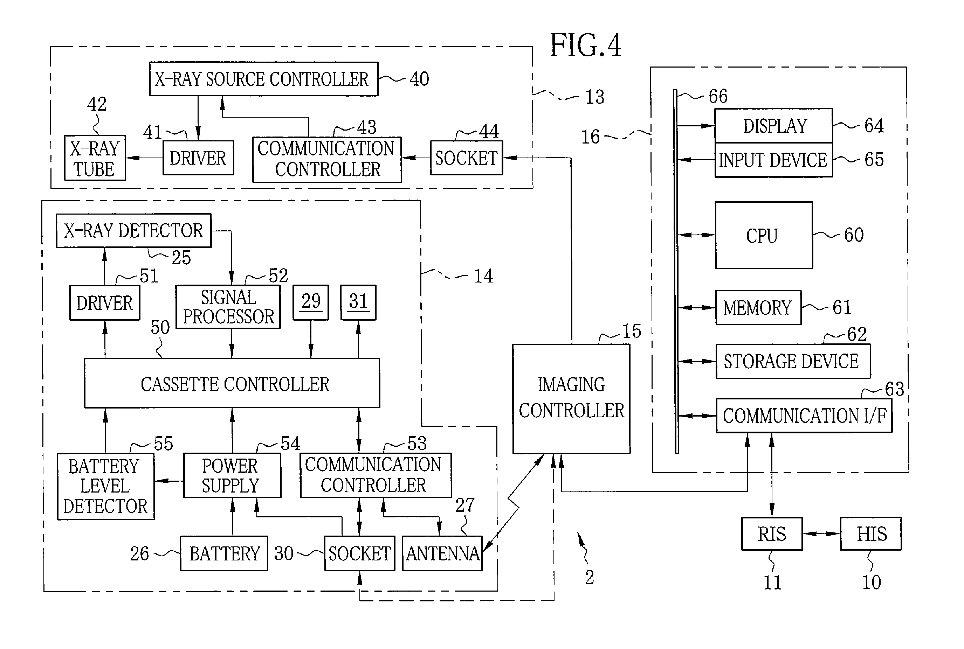

The console 16 of the X-ray imaging apparatus 2 produces an order list 21 from the orders 20 received from the HIS 10, and stores the order list 21 in a storage device 62 (see Fig. 4). As required, the console 16 reads out the order list 21 to display it on a display 64 (see Fig.4).

The inspection order 20 individually includes information items such as patient ID and patient' s name, identifying a patient to be subjected to the inspection, the order number of the inspection, body site to image, e.g. shoulder or knee front, progress status of the inspection, and notices or instructions that may be written by the doctor who orders the inspection. The inspection order may be submitted one for each patient to be inspected, or a set of inspection orders may be submitted at once with respect to a single patient. Although it is omitted from the drawings, the inspection order 20 may include an item for designating the type of inspection or the type of imaging apparatus or modality applied to the inspection. The HIS 10 and the RIS 11 sort the received order 20 according to the inspection type and the department that orders the inspection.

Referring back to Fig. 1, the X-ray imaging apparatus 2 consists of an X-ray source 13, a wirelessly communicable cassette 14 for detecting X-rays that was projected from the X-ray source 13 and traveled through a patient H under inspection and outputting image data corresponding to the detected X-rays, an imaging controller 15 for controlling the operation of the X-ray source 13 and the cassette 14, and the console 16 for inputting instructions to the imaging controller 15 and setting conditions for imaging, such as tube voltage, tube current, and exposure time of an X-ray tube 42 of the X-ray source 13.

The imaging controller 15 gives commands to the X-ray source 13 and the cassette 14 according to the imaging conditions and instructions received from the console 16, so that the X-ray source 13 and 14 can operate synchronously. The image data output as electric wave from the cassette 14 is fed to the console 16 via the imaging controller 15. The console 16, which may be a personal computer or a work station, processes the received image data, controls the display 64 to display the radiological images, or uploads the image data on a data storage device such as an image storing server.

The X-ray source 13 is connected to the imaging controller 15 through a cable, and is power-supplied from the imaging controller 15. The X-ray source 13 includes an X-ray tube 42 and a not-shown collimator that confines the projection field of the X-rays from the X-ray tube 42 into a rectangular shape. In an example, the X-ray source 13 is suspended from a ceiling of the X-ray room using a ceiling traveling mechanism such that the X-ray source 13 is movable in both vertical and horizontal directions. In addition, the X-ray source 13 can swing about a horizontal axis using a not-shown rotary mechanism. Thus the position of the X-ray source 13 is adjustable to the patient H according to its length and the aimed body site to image.

The cassette 14 is of substantially rectangular box-shaped. The cassette 14 is usable without any cable connection and may be installed anywhere, for example, located beneath the bed the patient lies on, or at another appropriate position corresponding to the aimed body site, e.g. shoulder or knee front.

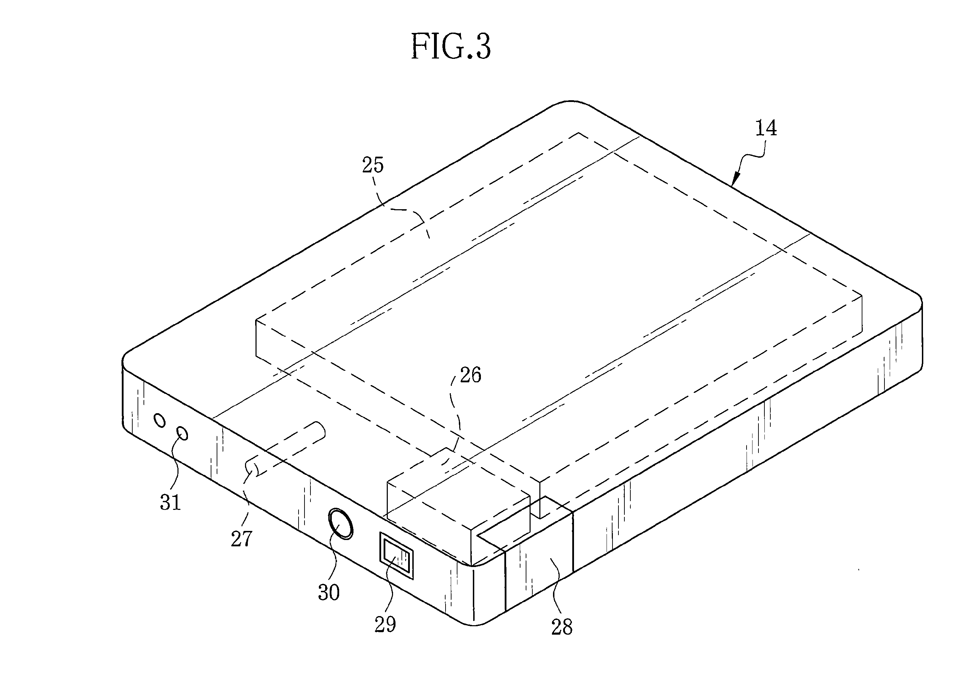

In Fig.3, the cassette 14 contains an X-ray detector 25. The X-ray detector 25 may for example be a flat panel detector (FPD) which has a matrix substrate on which a plurality of pixels are arranged in a matrix, each pixel consisting of a thin film transistor (TFT) and an X-ray sensor. The X-ray detector 25 accumulates electric charges in the X-ray sensors, and the amount of charge accumulated in each X-ray sensor corresponds to the amount of X-rays that falls on the sensor while the TFT is OFF. The charges are read out from the X-ray sensors while the TFT are turned ON. The read charges are converted to voltage signals through an integration amplifier of a signal processor 52 (see Fig.4). The voltage signals are analog-to-digital converted in the signal processor 52 to produce digital image data.

The cassette 14 also contains a battery 26 and an antenna 27. The battery 26 supplies power to activate components of the cassette 14. The battery 26 is relatively small so that it may be accommodated in the cassette 14 of a limited thickness. By opening a lid 28 on one side of the cassette 14, the battery 26 is removable from the cassette 14. The battery 26 is rechargeable by putting it in one of cradles 17 (see Fig.1). The antenna 27 wirelessly transmits and receives electric waves to and from the imaging controller 15.

The cassette 14 also has a power switch 29, a socket 30 and an indicator 31 on its another side. The socket 30 is for cable-connection with the imaging controller 15; a communication cable (not-shown) from the imaging controller 15 may be plugged in the socket 30. The communication cable may be employed when the wireless communication between the cassette 14 and the imaging controller 15 is impossible for some reasons, e.g. lack of remaining charge level. Through the communication cable, the cassette 14 is able to communicate with the imaging controller 15 and is power-supplied from the imaging controller 15. The indicator 31, which may for example be composed of multi-color LEDs, indicates power ON/OFF status of the cassette 14, charge level of the battery 26, wireless communicability and other information.

In Fig. 4, an X-ray source controller 40 controls the overall operations of components of the X-ray source 13. The X-ray source controller 40 controls the operation of the X-ray tube 42 through a driver 41 such that the X-ray tube 42 operates under the imaging conditions and at the operation timings as instructed by the imaging controller 15. The X-ray source controller 40 is also connected to a communication controller 43, which supplies the X-ray source controller 40 with various information and signals from the imaging controller 15, which are received through a socket 44.

A cassette controller 50 controls the overall operations of components of the cassette 14. The cassette controller 50 controls the operation of the X-ray detector 25 through a driver 51 such that the X-ray detector 25 operates at the operation timings as instructed by the imaging controller 15. The cassette controller 50 also receives image data from the signal processor 52.

A communication controller 53 is connected to the antenna 27 and the socket 30, to interface communication of various information and signals, including the image data, between the antenna 27 or the socket 30 and the cassette controller 50. In Fig.4, the connection between the socket 30 and the imaging controller 15 through communication cable is shown by a dashed line.

A power supply 54 supplies the respective components of the cassette 14 with the power from the battery 26 or the power received from the imaging controller 15 via the socket 30. The power supply 54 is connected to a battery level detector 55. The battery level detector 55 detects the battery level or remaining charge level of the battery 26 on the basis of the output from the power supply 54.

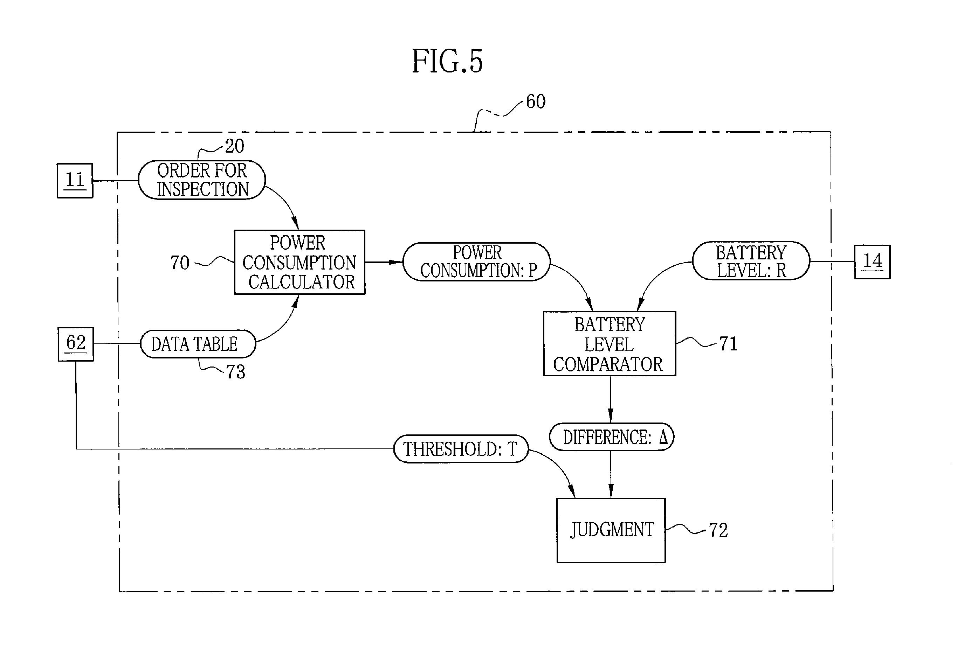

Specifically, the battery level detector 55 measures a discharge voltage from the battery 26, and analog-to-digital converts the measured value to output a digitized battery level or remaining charge level of the battery 26 to the cassette controller 50. Responding to a request from one of the consoles 16, the cassette controller 50 transmits the battery level "R" (see Fig.5) through the communication controller 53, the antenna 27 or the socket 30, and the imaging controller 15 to the console 16.

The console 16 includes CPU 60, memory 61, storage device 62, interface (I/F) 63, display 64, and input device 65 such as keyboard, mouse and microphone. These components are interconnected through a data bus 66.

The memory 61 is a work memory for the CPU 60 to use in executing processing. The storage device 62 is for example a hard disc drive. The storage device 62 stores OS and various applicable programs. The CPU 60 controls the respective components of the console 16 by loading an appropriate program from the storage device 62 to the memory 61 and executing processing according to the program. The CPU 60 also controls the respective components of the console 16 in response to operational input signals from the input device 65.

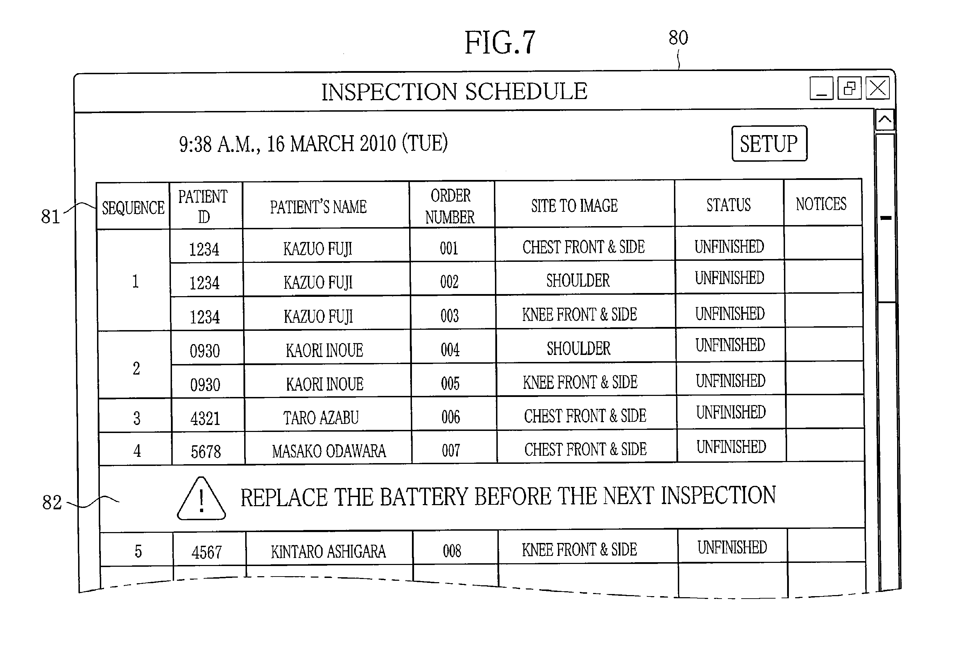

The communication I/F 63 may include LAN port, WAN port, or router, which is coupled to a communication cable that is connected to a network, such as the LAN 12 or the Internet. The communication I/F 63 interfaces data communication over the network. The display 64 may display various operational screens for entering instructions and conditions for imaging, a work list confirmation window 80 (see Fig.7) etc.

As the CPU 60 of the console 16 executes procedures written in the programs loaded from the storage device 62, the CPU 60 provides functions as illustrated in Fig.5.

In Fig.5, the CPU 60 of the console 16 functions as power consumption calculator 70, battery level calculator 71 and judgment section 72. The power consumption calculator 70 calculates an amount of power that the cassette 14 will consume for inspection or imaging of a patient with reference to the order list 21. The power consumption calculator 70 calculates the power consumption "p" for each individual patient each time an order 20 for inspection of a patient is received from the RIS 11 and added to the order list 21. The power consumption calculator 70 outputs the calculated power consumption "p" per one patient to the battery level calculator 71.

The power consumption "p" per one patient varies depending upon the number of X-ray images to be captured from the patient. Needless to say, the power consumption "p" increases with the number of captured images, and the remaining charge level of the battery 26 decreases more with the increased power consumption. The number of X-ray images to be captured may be generally predetermined according to the aimed body sites. Therefore, it is possible to prepare a data table 73 which shows relationships between body sites to inspect and estimated power consumption "p" (or the requisite number of images), and store the data table 73 previously in the storage device 62. Thus, the power consumption "p" per one patient may be estimated by the body site designated by the inspection order 20 with reference to the data table 73.

The battery level calculator 71 integrates the power consumption "p" per one patient as it is received from the power consumption calculator 70, to calculate a total sum "P" of the power consumption (P = ∑p), which represents an amount of power estimated to be consumed by the cassette 14 to execute all inspections having been requested at that point of time. Then the battery level calculator 71 subtracts the summed power consumption "P" from the battery level "R" to calculate a difference Δ (= R - P). The difference Δ represents an estimated remaining charge level of the battery 26, which would remain after all of the ordered inspections are finished. The battery level calculator 71 outputs the difference Δ to the judgment section 72.

The judgment section 72 compares the difference Δ with a threshold T which represents a minimum charge level below which the battery 26 cannot work. The threshold T is previously stored in the storage device 62. Preferably, the threshold T is set at a level slightly higher than an actual minimum battery level. When the difference Δ is not less than the threshold T (difference Δ ≥ T), the judgment section 72 judges that the current battery level "R" can cover the demand for all inspections ordered at present. On the contrary, when the difference Δ is less than the threshold T (difference Δ < T), the judgment section 72 judges that the current battery level "R" cannot cover the demand for all of the inspections ordered at present.

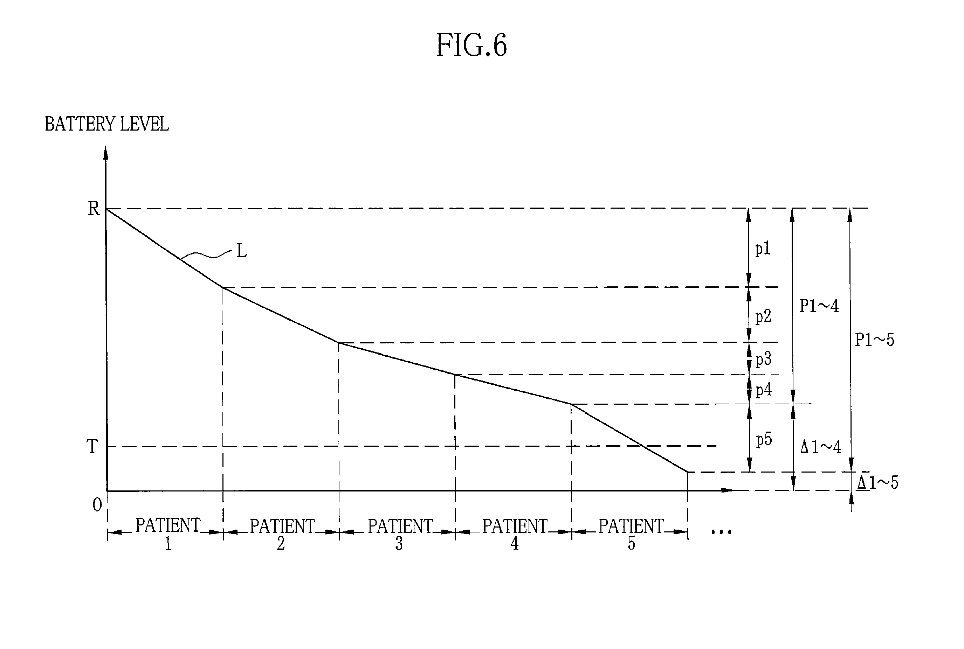

A particular example will be described with reference to Fig. 6, wherein vertical and horizontal axes represent the battery level and the number of patients respectively. As a result of calculation at the power consumption calculator 70, the battery level is estimated to go down from the current level R in a manner as shown by a bent line L along with the progress of inspections on patients #1, #2 ....

In the example shown in Fig. 6, it is assumed that the console 16 has already received four orders 20 for inspections on patients #1 to #4, and has just received a new order 20 for an inspection on a patient #5. Strictly speaking, if there is a time lag from the reception of the inspection order 20 for the fourth patient #4 to the reception of the newly added inspection order 20 for the fifth patient #5, and the power of the battery 26 is consumed during this time lag, the current battery level R will change correspondingly. However, for the sake of simplicity, the following description assumes that the battery level R remains unchanged between the receptions of the fourth and fifth inspection orders for the patients #4 and #5.

Before the inspection order for the fifth patient #5 is added to the order list 21, the difference Δ1-4 between the battery level R and the sum P1-4 of the power consumption amounts p1 to p4 is still above the threshold T. Therefore, the judgment section 72 judges that the current battery level R is sufficient for powering the cassette 14 to complete all inspections on the order list 21.

When the fifth inspection order for the patient #5 is added to the order list 21, the power consumption calculator 70 calculates an estimated power consumption amount p5 of the cassette 14 necessary for the inspection on the patient #5. Then the battery level calculator 71 calculates a difference Δ1-5 between the battery level R and a sum P1-5 of the power consumption amounts p1 to p5. As shown in Fig.6, the difference A1-5 is below the threshold T. It means that the charge level of the battery 26 will get too low to continue the inspection on the patient #5 without replacing the battery 26 before starting the inspection on the patient #5. Accordingly, the judgment section 72 judges that the current battery level R is insufficient for powering the cassette 14 to complete all inspections ordered at present.

When the judgment section 72 judges that the current battery level R is insufficient for carrying out all of the ordered inspections, the console 16 displays a work list confirmation window 80 on the display 64, as shown for example in Fig.7, in order to avoid interruption of the inspection due to battery rundown.

The work list confirmation window 80 has a list display section 81 and a message display section 82. The list display section 81 shows basically the same contents as the order list 21, but is compiled into a display style that facilitates recognizing the contents registered in the order list 21, e.g. with an additional column of serial numbers indicating the sequence of executing the inspections, which principally corresponds to the order of receipt. The list display section 81 may be scrollable by operating the mouse or the like.

In the preferred embodiment shown in Fig.7, the message display section 82 is located between a line of displaying information on the inspection until which the battery level R will not be less than the threshold T, and a line of displaying information on the inspection during which the battery level R will be less than the threshold T. Corresponding to the example shown in Fig.6, the message display section 82 is displayed between a line for the patient #4 and the line for the patient #5. The message display section 82 presents a message prompting to replace the battery before the next inspection. So long as the judgment section 72 judges that the current battery level R is sufficient for powering all of the inspections on the order list 21, that is, until the inspection order for the patient #5 being added in the case of Fig. 6, the message display section 82 does not appear in the work list confirmation window 80.

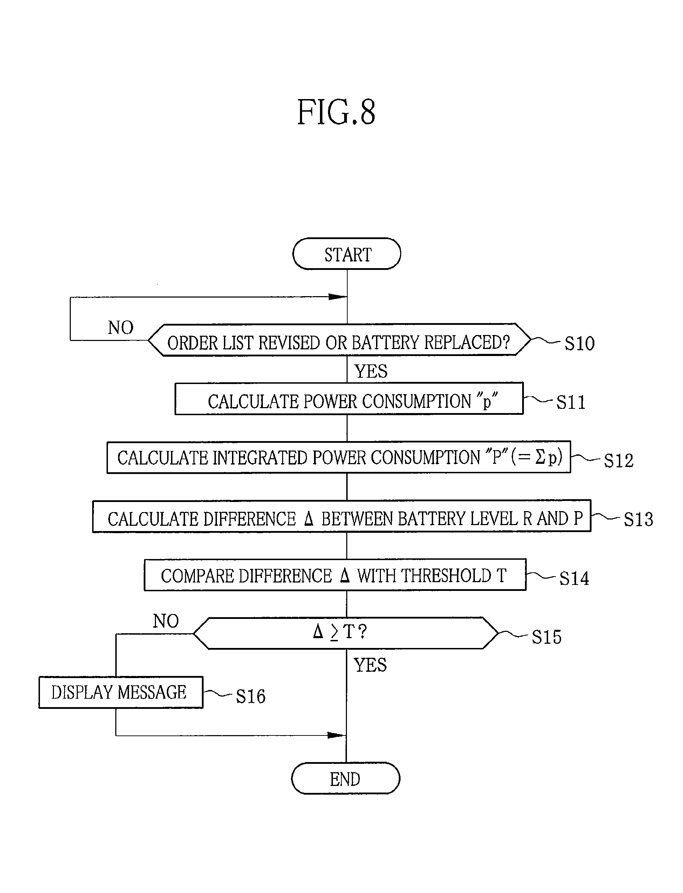

Now the operation sequence of the X-ray imaging apparatus 2 configured as above will be described with reference to the flowchart of Fig.8.

When a doctor in a diagnosis and treatment department enters an order 20 for an inspection of a patient at a terminal of the HIS 10, the order 20 is sent through the HIS 10 and the RIS 11 to one console 16. In response to the inspection order 20, the console 16 revises the order list 21 in the storage device 62 (YES in step S10).

Upon the order list 21 being revised, the CPU 60 of the console 16 requests the cassette 14 to transmit the current battery level R. Simultaneously, the power consumption calculator 70 calculates an amount of power consumption "p" that the cassette 14 will consume for the ordered inspection (step S11). The battery level calculator 71 integrates the power consumption amounts "p" for the individual inspections which have been requested and received at present, to estimate the total amount of power consumption "P" that will be necessary for the cassette 14 to execute a11 of these inspections (step S12). Thereafter, the battery level calculator 71 calculates a difference Δ between the power consumption P and the battery level R (step S13).

Then the judgment section 72 compares the difference Δ with a predetermined threshold T of the minimum battery level (step S14). When the difference Δ is not less than the threshold T (YES in step S15), the judgment section 72 judges that the current battery level "R" is sufficient for all the inspections ordered at present. In that case, the work list confirmation window 80 merely displays the list display section 81.

On the contrary, When the difference Δ is less than the threshold T (NO in step S15), the judgment section 72 judges that the current battery level "R" is insufficient for the cassette 14 to accomplish all of the inspections ordered at present. In that case, the message display section 82 appears on the work list confirmation window 80 in association with the list display section 81, prompting a radiologist to replace the battery 26 before starting the inspection that is listed next to the message display section 82 in the list display section 81. Thus, the cassette 14 can avoid running out of power in the middle of an inspection. Making the judgment based on the latest order list 21, the CPU 60 can accurately forecast the time to replace the battery 26 of the cassette 14.

In practice, the console 16 tends to receive a plurality of orders 20 for inspections of different patients at once in a limited time slot such as immediately after the start time or the lunch hour of the hospital. In that case, the judgment section 72 may judge that the current battery level R of the cassette 14 cannot cover the demand for all of the inspections as soon as the orders are received in a unit. Then the CPU 60 can control the display 64 to display the message display section 82 with the list display section 81 from the begging of the work list confirmation window 80.

When the battery 26 is replaced, the CPU 60 should conduct the above-described battery level judgment (steps S11 to S16) independently of whether a new order 20 for inspection is added to the order list 21. The cassette 14 may detect the replacement of the battery 26 for example by detecting the opening-and-closing of the lid 28 or removable-and-insertion of batteries 26 using a limit switch or the like. Upon detecting the replacement of the battery 26, the cassette 14 sends a cue to the console 16, so that the console 16 may restart the battery level judgment.

If any of the received orders 20 is canceled, the order list 21 will be revised and the console 16 also restarts the battery level judgment according to the change in power consumption P, wherein the cancellation may be of a set of orders for imaging different sites of a patient or of one or more of orders for a patient. As described above, the disclosed battery level managing system makes the battery level judgment time after time according to many variable factors, including the current battery level R and the number of requested inspections, which can vary momentarily. Thus, the disclosed system can always provide updated information on the time to replace the battery.

Although the above embodiment has been described with respect to a situation where the difference Δ between the current battery level R and the sum P of estimated power consumption by the cassette 14 for all of the currently requested inspections is calculated and compared with the threshold T when a new order for inspection is added to the order list 21 before starting the first inspection on the list, the calculation and comparison of the difference Δ with the threshold T may also be executed each time an ordered inspection is finished or a requisite number of images have been captured from a patient, and hence the order list 21 is revised.

In the above embodiment, the power consumption "p" of the cassette 14 for one patient is calculated based on the body sites to inspect, which designate the number of images to be captured. However, the number of images captured from one patient may vary from the number predetermined by the body sites to inspect. This is because the radiologist can fail to capture an adequate image and retry to capture another image for compensation, or the radiologist may decide to capture more images than requested. In that case, the cassette 14 will consume more power than the value "p" predicted based on the estimated number of images to capture. Hence the integrated power consumption "P" and the difference Δ will differ from the actual values. In addition, the cassette consumes power during the standby periods, i.e. while the patients under inspection are interchanging and in the intermission between the imaging of one body site and another.

Accordingly, the power consumption "p" calculated based on the number of images predetermined by the individual body sites may differ from the actual power consumption. Therefore, the battery level calculator 71 preferably adds a margin to the power consumption "p" as calculated by the power consumption calculator 70, so as to take account of the power consumption during the standby periods and the potential power consumption for one additional image per one patient. It is to be noted that the threshold T may also be predetermined so as to take account of the power consumption during the standby periods and the potential power consumption for additional images which might be captured in compensation for defective images or for other reasons.

In practice, power consumption per one X-ray image differs from each other between different types of cassettes. In addition, rechargeable batteries generally tend to run down the faster the more number of times they have been recharged. In view of this, the estimated power consumption "p" may be corrected so as to take the cassette type and the usage of the battery into consideration in addition to the number of images predetermined by the body sites, as well as the power consumption for compensative images and during the standby periods.

Specifically, a plurality of cassettes and their batteries may individually hold their own ID data in EEPROMs or RFID tags which are built in their housings, so that the RIS may manage information about the cassette types and the usage of the batteries in a manner as will be described in detail in a second embodiment. The respective IDs may be set up in the course of manufacturing and shipping the cassettes and batteries, or may be set up appropriately in hospitals. Information items about battery usage may include how often the battery has been recharged, how long the battery has been used, how long the battery has been charged, when the battery was first put into service, etc. Among these items, the recharged frequency and time may be detected by the cradles 17 and transmitted to the RIS 11 through the LAN 12.

The cassette may read the battery ID to transmit it together with the cassette ID to the console. The console refers these IDs to the RIS to retrieve information about the cassette type and the usage of the battery. The console then refers to data table storing correction values for correcting the power consumption "p" according to the cassette type and the usage of the battery corrects, to correct the power consumption "p" with appropriate correction values. Correcting the previously stored power consumption "p" so as to take such factors into account that cause variations in actual power consumption will make the estimated value "p" closer to the actual power consumption, and hence improve the accuracy of calculating the remaining battery level, i.e. the difference Δ.

Alternatively, actual power consumption may be measured aside from the estimated power consumption "p", to rewrite the data table 73 to replace the power consumption "p" with the actual power consumption. For example, actual power consumption measured at each imaging of patient's shoulder may be accumulated and averaged, to replace the power consumption for the shoulder stored in the data table with the actual average power consumption. Continually revising the power consumption data table in this manner will make the power consumption data more reflective of actual power consumption, resulting in improving the calculation accuracy of the remaining charge level.

The battery level judgment may also be executed after each execution of inspection on a patient. Thereby, the difference between the calculated power consumption and the actual power consumption will be absorbed. Displaying the time to replace the battery 26 at each completion of inspection on a patient will make the most of the waiting time for the next patient, permitting the operating radiologist to replace the battery 26 well in advance.

The message prompting the battery replacement may be displayed on the work list confirmation window as soon as the judgment section determines that the current battery level R is insufficient for accomplishing all of the presently ordered inspections, like in the above embodiment, or may also be displayed at every intermission between inspections on one patient and another. In a word, the work list confirmation window may be displayed any time before the time to replace the battery, i.e., before the start of an inspection during which the currently used battery will run down.

Concurrently with displaying the message on the work list confirmation window, the console may give an alarm from its speaker so as to call attention of the operating radiologist to the time to replace the battery. Alternatively, the radiologist may wear a wrist band which is wirelessly communicable with the console and configured to give an audio alarm or vibrations to inform of the time to replace the battery.

The message display section may be displayed as a separate window from the work list confirmation window. The message contained in such an independent message display window should clearly indicate the time to replace the battery; the message may be like "Replace the battery before the fifth inspection". It is also possible to provide a cassette housing with a display device, such as an LCD panel, to display a message predicting the time to replace the battery.

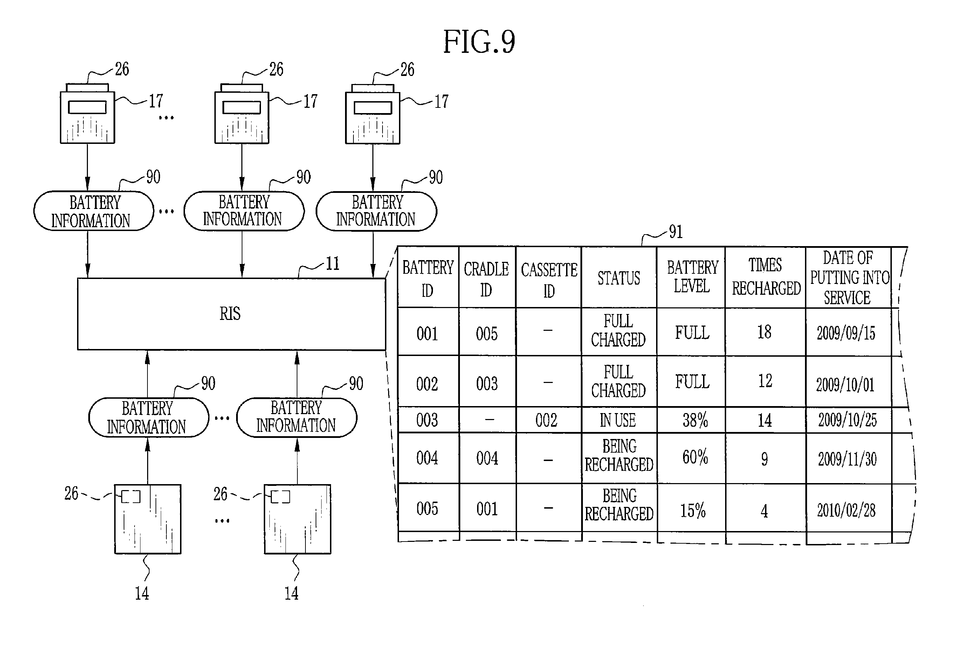

Now another embodiment of the present invention will be described with reference to Fig.9, wherein RIS 11 acquires battery information 90 from multiple batteries 26 to monitor the usage of these batteries 26 and give useful assistance for the battery replacement.

In the embodiment of Fig. 9, the RIS 11 collects the battery information 90 about those batteries 26 which are loaded in the cradles 17, hereinafter referred to as spare batteries 26, as well as those just used in the cassettes 14, through transmission over the LAN 12, and creates a battery information list 91 from the collected battery information 90. The RIS 11 stores and manages the battery information list 91.

The battery information 90 includes a battery ID, status data, battery level, the number of times having been recharged, and the date of putting into service, etc. The cradle 17 may be provided with a similar battery level detector to the above described battery level detector 55. When the battery information 90 is transmitted from the individual cassette 14 or cradle 17 to the RIS 11, a corresponding cassette ID or cradle ID is added to the battery information 90.

The battery information list 91 shown in Fig.9 shows that a battery 26 with a battery ID "003" is used in a cassette 14 with a cassette ID "002", that spare batteries with battery IDs "001" and "002" are fully charged at cradles with cradle IDs "005" and "003" respectively, and that spare batteries with battery IDs "004" and "005" are being charged at cradles with cradle IDs "004" and "001" respectively.

When the judgment section 72 judges that the battery level R of the battery 26 loaded in the currently used cassette 14 is too low to power the cassette 14 throughout the presently ordered inspections, the console 16 sends an alert of the necessity and time to replace the battery 26 to the RIS 11 along with battery information 90 of the currently used battery 26 and the calculated power consumption values "p". Then the RIS 11 refers to the battery information list 91 to select an available one of the spare batteries 26, and sends back the battery information 90 of the selected battery 26 to the console 16.

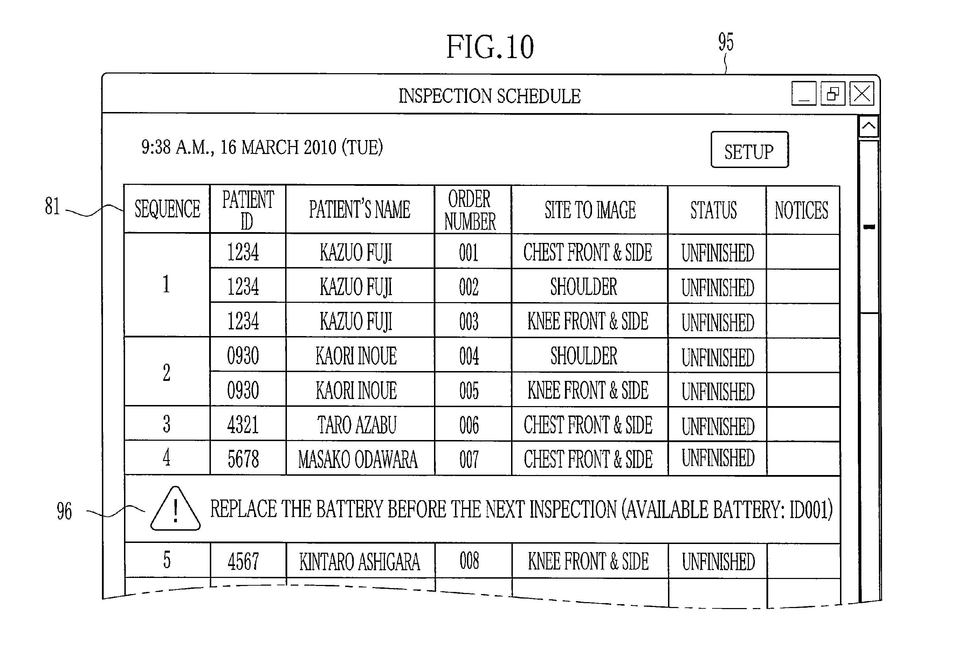

Thus the console 16 displays information on the available battery 26 in addition to a message informing of the necessity and time to replace the battery 26. For example, as shown in Fig.10, a message display section 96 of a work list confirmation window 95 may display a message "Replace the battery before the next inspection" and information on the available spare battery "available battery: ID001". The message display section 96 may be displayed as a separate window from the work list confirmation window 95, indicating the time of replacement beside the prompting message.

The available spare battery may be selected from fully charged ones, i.e. batteries of ID "001" or "002" in the example shown in Fig. 9. Alternatively, the available spare battery may be selected from the most often recharged battery or the oldest one that has been used for the longest time, in order to retard deterioration of new batteries. On the contrary, it may also be possible to select preferentially the fewest recharged battery or the newest as the available battery for the purpose of averaging the degree of deterioration among all batteries 26.

In practice, it is usual to charge all cassette batteries at once in the closing time of the hospital or during the lunch hour regardless of their battery levels, in order to prepare for inspections on the next day or in the afternoon. However, recharging a battery before it is fully discharged will lead to lower the charging efficiency and accelerate deterioration in comparison with the case where the battery is recharged after being fully discharged. In view of this, it is preferable to select the available spare battery considering whether it will be used up by the closing time or the lunch hour in the hospital, in addition to or instead of the above recited criteria such as whether it has been charged up, how often it has been recharged, how long it has been used, etc.

For instance, if the inspection, during which the judgment section 72 determines the currently used battery will run down, is a last one that is going to be carried out immediately before the closing time or the lunch hour in the hospital, it is preferable to select such a spare battery 26 that has a battery level closest to the sum of power consumption "p" necessary for the last inspection (i.e. p5 in the illustrated example) and the given threshold T, from among the spare batteries 26. By carrying out the last inspection with the spare battery 26 thus selected, this battery 26 will be almost fully discharged at the end of the last inspection, i.e. immediately before the closing time or the lunch hour of the hospital.

It would be understood from the above description that the minimum requirement for the battery to be selected from among the spare batteries is that its battery level should be equal to or more than the sum of power consumption "p" for the inspection, for which the battery level R of the currently used battery is judged to be insufficient, and the threshold T.

It should be noted that a message recommending to replace the battery may be given each time the order list is revised, indicating such a battery as an alternative that has a sufficient battery level enough to accomplish all of the presently ordered inspections, instead of a message indicating a battery of a battery level suitable for carrying out those of the ordered inspections which are after the last inspection that can be dealt with using the battery presently loaded in the cassette.

Taking the case of Fig.6 for example, when the order for inspection on the patient #5 is received, the battery level of the presently loaded battery is judged to be insufficient for the inspection of the patient #5 because the calculated difference Δ1-5 gets smaller than the threshold T. At this point, if the battery is replaced with another having a sufficient battery level R that is not less than the sum of the power consumption P1-5 and the threshold T, which can be selected from the battery information list, the following inspections including one on the patient #5 may be accomplished with the newly loaded battery.

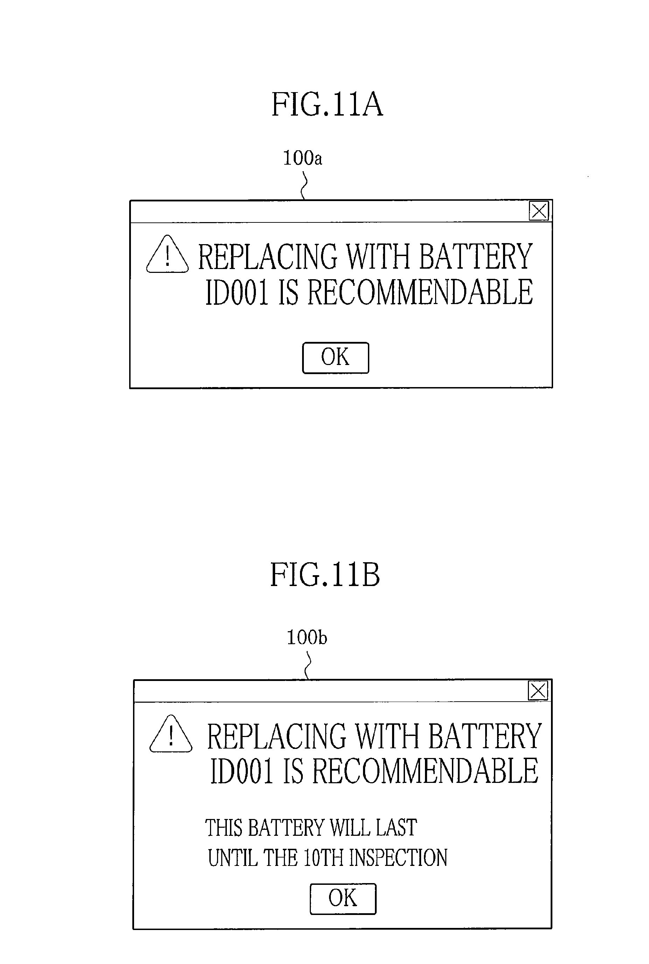

In that case, a message window 100a indicating a recommendable alternative battery, as shown in Fig.11A, may be popped up on the display 64 in response to the battery level judgment. Informing of the recommendable alternative battery well in advance to the time limit to replace the battery of the cassette advantageously enables replacing the battery at any appropriate time, e.g. in a spare time resulted from cancellation of an inspection or a delay of arrival of a patient.

In case where there is only one spare battery, it is preferable to display the message window 100a as soon as the charge level of the spare battery reaches a level equal to or higher than the sum of the integrated power consumption P (=∑p) and the threshold T.

In medical inspections within a hospital, movements of radiologists and patients are complicated and have variable flow lines from one hospital to another. Therefore, it is difficult to predict systematically the individual movements of these persons, including the patient's delay of arrival. Accordingly, it is difficult for the system to determine the optimum timing of battery replacement. However, by informing of the available alternative battery in advance to the time limit, e.g. as soon as the charge level of the spare battery reaches a sufficient level, e.g. a level equal to or higher than the sum of the power consumption P and the threshold T, the operator or radiologist can replace the battery at an appropriate spare time during the following inspections. Consequently, the disclosed system may give more chances for battery replacement and thus boost the convenience.

Even after the message window 100a is displayed, it may be possible to check if the charge level of the spare battery is equal to or more than the sum of the power consumption P and the threshold T once again, for example, when an inspection order is added to the order list and thus the estimated power consumption P is updated. Then, if it is determined that the charge level of the spare battery is lower than the revised sum P+T, the message window 100a will be closed. This way, the system can keep up with the latest condition of the spare batteries as well as the battery loaded in the cassettes, thereby to assist the radiologists to replace the batteries at appropriate timings.

Instead of a message pop-up window like the window 100a, a message informing of an available spare battery may be displayed in a corner of the work list confirmation window 95 of Fig.10. Where there are many spare batteries, messages about available spare batteries would be frequently displayed on the work list confirmation window 95. In that case, the message may preferably be displayed in a margin outside the list display section 81.

Preferably, the message window 100a is displayed immediately after finishing each set of inspections on one patient which are carried out before a time limit to replace the battery. Thus, the battery may be replaced with the spare battery according to the message in the window 100a, making use of a vacant time between the end of inspection on one patient and the start of inspection on another patient.

It is also preferable to inform not only an available spare battery but also the lasting time of the available spare battery, like a message window 100b as shown in Fig.11B. In that case, the console 16 transmits the power consumption amounts p for the individual inspections to the RIS 11, to detect an inspection during which the difference Δ between the integrated value P of the power consumption p and the current charge level R of the spare battery would go below the threshold T. Then an inspection preceding to the detected inspection can be displayed as the time limit for the spare battery until which it can effectively supply the cassette. Predicting the durability of a spare battery before it is loaded in the cassette will facilitate planning the following inspections after the battery replacement, and hence smoothing the execution of the inspections.

It is possible to inform of the durability of a spare battery not only in the case where the availability of the spare battery is displayed in advance to the time limit to replace the presently used battery, but also in the case where the available spare battery is indicated at the time to replace the presently used battery. The charge level of the spare battery can further increase since it was selected as the replaceable battery, as the spare battery is kept being charged in the cradle 17 until it is actually replaced with the used battery and loaded in the cassette. Therefore, where the available spare battery is selected in advance to the time limit to replace the presently used battery, it is preferable to predict the charge level that the individual spare battery will reach by the time of replacing the battery, and select the available battery and estimate its durability on the basis of the predicted charge level. Because the charging property of each battery varies with its deterioration degree, it is preferable to consider the deterioration degree when predicting the charge level of the spare battery.

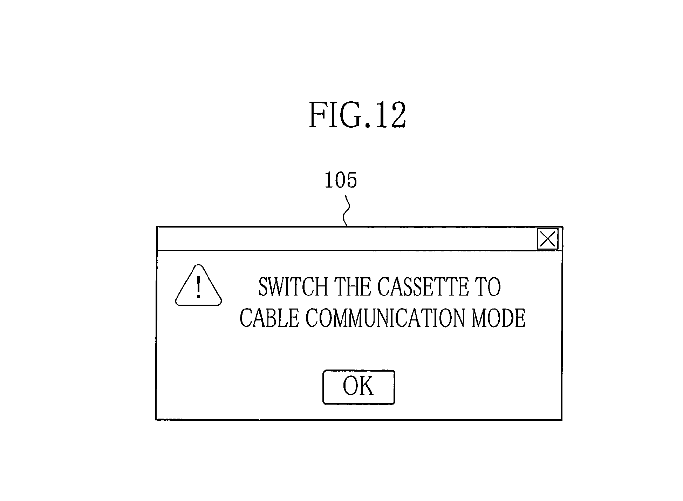

If none of the spare batteries 29 have the charge level necessary for selecting as the available battery, a message window 105 noticing the necessity to switch the cassette to wired communication may be popped up on the display 64, as shown for example in Fig.12. Thus, the operator can quickly switch the cassette to the wired communication mode, avoiding replacing the battery with one having insufficient charge level.

In the above embodiment, the time available for replacing the battery is detected and notified with reference to the charge levels of the spare batteries and the estimated power consumption of the cassette when the remaining charge level of the presently used battery goes below the level necessary for carrying out all inspections ordered at present. In an alternative embodiment, the time available for replacing the battery may be detected and notified merely on the basis of the charge levels of the spare batteries and the estimated power consumption of the cassette regardless of the remaining charge level of the presently used battery. Specifically, the message window 100a may be displayed as soon as the charge level of the spare battery reaches a level equal to or higher than the sum of the power consumption P and the threshold T, independently of the presently used battery. Thereby, the operator or radiologist can get more chances to replace the battery with improved convenience.

In a place where multiple X-ray imaging apparatuses are installed, it is possible to monitor the battery levels of the respective cassettes used in these imaging apparatuses and allot inspection orders from the RIS to the consoles of these imaging apparatuses so that the cassettes of these imaging apparatuses will not run out of battery. More specifically, such an inspection that will consume an electric power p of the battery that is substantially equal to or slightly less than a value obtained by subtracting the threshold T from a difference Δ between a remaining battery level R and an integrated power consumption P. In other words, the inspection order may be allotted such that the difference Δ will be approximately equal to the threshold T when the power consumption p for the allotted inspection is integrated into the value P.

Although the batteries are removed from the cassettes, to be recharged by the cradles in the above embodiments, cassettes may be configured such that they can be put in cradles to recharge their batteries without separating from the cassettes.

In the above embodiments, the power consumption calculator, the battery level calculator and the judgment section are developed in the CPU of the console. The present invention is not limited to this configuration, but these functional sections may be provided in the RIS, or in a specific battery managing apparatus other than the console and the RIS. It is also possible to transmit information about discharge voltage of each battery from the cassette to the console so as to detect the battery level or remaining charge level of the battery. Although the consoles and the cradles are connected through the LAN in the illustrated embodiment, the cradles may be directly connected to the consoles.

It should be understood that the embodiments of the present invention have been disclosed for illustrative purposes only. Those skilled in the art will appreciate that various modifications, additions and substitutions are possible without departing from the scope and spirit of the invention as disclosed in the accompanying claims.

## Claims

1. A battery managing apparatus for a wirelessly communicable cassette (14) for use in medical inspections, comprising:
a first battery level detector (55) that detects a current charge level (R) of a battery presently used in the cassette;
a power consumption calculator (70) that calculates an estimated amount of power consumption (p) by the cassette for each of received orders for inspections;
a battery level calculator (71) that calculates an estimated remaining charge level (Δ) of the presently used battery, which will remain at the completion of each of the ordered inspections, on the basis of the current charge level (R) detected by said first battery level detector (55) and the power consumption (p) calculated by said power consumption calculator (70) ;
a first judgment device (72) that determines on the basis of the estimated remaining charge level (Δ) the last inspection among the ordered inspections, until which the presently used battery can effectively supply the cassette; and
a first display device (82) that displays a time limit to replace the presently used battery at latest by the end of the last inspection until which the presently used battery can effectively supply the cassette.

2. The battery managing apparatus as recited in claim 1, wherein said first battery level detector (55), said power consumption calculator (70), said battery level calculator (71) and said first judgment device (72) are activated when the orders for inspections are revised.

3. The battery managing apparatus as recited in claim 1 or 2, wherein said first battery level detector (55), said power consumption calculator (70), said battery level calculator (71) and said first judgment device (72) are activated every time an inspection on a patient is finished.

4. The battery managing apparatus as recited in either of claims 1 to 3, wherein said first battery level detector (55), said power consumption calculator (70), said battery level calculator (71) and said first judgment device (72) are activated every time the battery of the cassette is replaced.

5. The battery level managing apparatus as recited in either of claims 1 to 4, wherein said battery level calculator (71) calculates the estimated remaining charge level (Δ) of the presently used battery, which will remain at the completion of each of the ordered inspections, by subtracting an integrated value (P = ∑p) of the power consumption amounts (p) calculated by said power consumption calculator (70) from the current charge level (R) of the presently used battery; and
said first judgment device (72) compares the estimated remaining charge level (Δ) with a threshold (T) representative of a minimum effective battery level and, where the estimated remaining charge level (Δ) gets less than the threshold (T), determines that with the detected current charge level the presently used battery will not be able to supply for the inspection about which the remaining charge level (Δ) is estimated.

6. The battery level managing apparatus as recited in either of claims 1 to 5, wherein said power consumption calculator (70) calculates the power consumption (p) for each inspection, taking account of at least one of power consumed for an additional imaging operation, power consumed during standby status of the cassette, type of the cassette, or usage of the battery.

7. The battery managing apparatus as recited in any one of claims 1 to 6, further comprising:
a second battery level detector (17) that detects a current charge level of a spare battery other than the battery presently used in the cassette;
a second judgment device (72?) that determines based on the current charge level of the spare battery and the power consumption (p) calculated by said power consumption calculator (70) whether the spare battery is available for replacement with the presently used battery; and
a second display device (96, 100a) that displays information about an available spare battery as a result of judgment by said second judgment device, at latest by the end of the last inspection until which the presently used battery can supply the cassette effectively.

8. The battery managing apparatus as recited in claim 7,
wherein
said second judgment device determines that the spare battery is available for replacement with the presently used battery when the current charge level of the spare battery gets equal to or higher than a sum of an integrated value (P = ∑p) of the power consumption (p) necessary for each inspection as calculated by said power consumption calculator (70) and the threshold (T) representative of the minimum effective battery level; and
said second display device displays the information about the available spare battery when said second judgment device determines that the spare battery is available for replacement with the presently used battery.

9. The battery managing apparatus as recited in claim 7, wherein said second judgment device determines the spare battery to be available for replacement when the current charge level of the spare battery is approximately equal to a sum of a power consumption amount (p) necessary for one inspection during which the presently used battery will rundown and the threshold (T) representative of the minimum effective battery level.

10. The battery managing apparatus as recited in any one of claims 7 to 9, wherein there are a plurality of spare batteries, and said second judgment device determines a spare battery as available for replacement, taking account of usage of the respective spare batteries.

11. The battery managing apparatus as recited in any one of claims 7 to 10, wherein said second display device displays the information about the available spare battery determined by said second judgment device immediately after the end of each inspection on one patient.

12. The battery managing apparatus as recited in any one of claims 7 to 11, wherein
said battery level calculator (71) calculates an estimated remaining charge level of the available spare battery determined by said second judgment device, which will remain at the completion of each of the ordered inspections when the available spare battery is used therefor, on the basis of the current charge level of the available spare battery and the power consumption (p) calculated by said power consumption calculator (70); and
said first judgment device (72) determines on the basis of the estimated remaining charge level of the available spare battery the last inspection until which the available spare battery will be able to supply the cassette effectively; and wherein
said battery managing apparatus further comprises a third display device (100b) that displays information about the last inspection until which the available spare battery will be able to supply the cassette effectively.

13. The battery managing apparatus as recited in any one of claims 7 to 12, further comprising a fourth display device (105) that displays a message prompting to switch the cassette to wired communication when there is no spare battery available.

14. A method of managing batteries used for supplying wirelessly communicable cassettes comprising steps of:
detecting a current charge level of a battery presently used in the cassette;
calculating an estimated amount of power consumption by the cassette for each of received orders for inspections;
calculating an estimated remaining charge level of the presently used battery, which will remain at the completion of each of the ordered inspections, on the basis of the current charge level of the presently used battery and the estimated power consumption calculated for each inspection;
determining on the basis of the estimated remaining charge level the last inspection among the ordered inspections, until which the presently used battery can effectively supply the cassette; and
displaying a time limit to replace the presently used battery at latest by the end of the last inspection until which the presently used battery can effectively supply the cassette.

15. A battery managing system for wirelessly communicable cassettes, said system comprising at least a wirelessly communicable cassette (14) for use in inspections and a battery managing apparatus for managing batteries used for supplying said wirelessly communicable cassette, wherein said battery managing apparatus comprises:
a battery level detector (55) that detects a current charge level (R) of a battery presently used in the cassette;
a power consumption calculator (70) that calculates an estimated amount of power consumption (p) by the cassette for each of received orders for inspections;
a battery level calculator (71) that calculates an estimated remaining charge level (Δ) of the presently used battery, which will remain at the completion of each of the ordered inspections, on the basis of the current charge level (R) detected by said battery level detector (55) and the power consumption (p) calculated by said power consumption calculator (70) ;
a judgment device (72) that determines on the basis of the estimated remaining charge level (Δ) the last inspection among the ordered inspections, until which the presently used battery can effectively supply the cassette; and
a display device (82) that displays a time limit to replace the presently used battery at latest by the end of the last inspection until which the presently used battery can effectively supply the cassette.
